# EUROPEAN PATENT APPLICATION

(11) **EP 2 471 466 A2**
(43) Date of publication of application: **04.07.2012**
(21) Application number: 10812351.4
(22) Date of filing: 30.08.2010
(51) Int. Cl.: A61B 17/04, A61B 17/068

(54) **SUTURING INSTRUMENT HAVING A FIXING MEANS**

(30) Priority: 28.08.2009 KR 20090080295
(71) Applicant: Rimscience Co., Ltd., Seoul 156-881 (KR)
(72) Inventor: YOON, Sang Jin, Seoul 137-925 (KR)
(74) Representative: Zardi, Marco
(86) International application number: PCT/KR2010/005852
(87) International publication number: WO 2011/025338

(57) **Abstract**

Provided is a suture apparatus having a fixing means. In accordance with one aspect of the present invention, there is provided a suture apparatus including: a support, a needle driving unit, and a surgical needle, wherein the surgical needle is accommodated in the needle driving unit to move with respect to the support, and a first suture from the surgical needle and a second suture from the support are entangled according to the movement, wherein the suture apparatus further includes a fixing unit for fixing a tissue to be sutured to the support and releasing the fixation of the tissue.

## Description

### TECHNICAL FIELD

The present invention relates to a suture apparatus having a fixing unit.

### BACKGROUND ART

In a surgical operation, it is essential to suture various tissues, such as a serosa, a muscle, a fascia, a skin, a blood vessel, or the like of a human body. Conventionally, such suturing was totally dependent upon an operator's manual manipulation or a surgical robot which merely serves to aid the operator's manual manipulation.

### DISCLOSURE

### TECHNICAL PROBLEM

In order to improve the foregoing situations, the inventor of the present application, who invented a novel surgical suture apparatus, filed a related application under Korean Patent Application No. 2009-37128 which was already granted (the entire disclosure of which should be considered to be incorporated herein by reference).

In addition, the inventor of this application proposes a novel technique which may be applied to the surgical suture apparatus or a similar suture apparatus to perform suturing more efficiently, as follows.

### TECHNICAL SOLUTION

It is an object of the present invention to solve all of the aforementioned problems.

It is another object of the present invention to provide a suture apparatus having a novel fixing means for ensuring efficient suturing.

### ADVANTAGEOUS EFFECTS

In accordance with the present invention, a suture apparatus having a novel fixing means for ensuring efficient suturing can be provided.

### DESCRIPTION OF DRAWINGS

The above objects and features of the present invention will become apparent from the following description of the preferred embodiments given in conjunction with the accompanying drawings, in which:
Fig. 1 is a drawing illustrating the overall configuration of a suture apparatus in accordance with an embodiment of the present invention.
Fig. 2 is a drawing illustrating the configuration of a suturing part in accordance with an embodiment of the present invention.
Fig. 3 is a drawing schematically showing an example of a suturing principle of a suture apparatus in accordance with the present invention.
Fig. 4 is a drawing illustrating the overall configuration of a suture apparatus in accordance with another embodiment of the present invention.
Fig. 5 is a drawing illustrating the configuration of a fixing unit in accordance with an embodiment of the present invention.
Fig. 6 is a drawing illustrating a spring in accordance with an embodiment of the present invention.
Fig. 7 is a drawing illustrating the configuration of a pressing plate in accordance with various embodiments of the present invention.

### BEST MODE

Several representative configurations for achieving the aforementioned objects of the present invention are presented as follows.

In accordance with one aspect of the present invention, there is provided a suture apparatus comprising: a support, a needle driving unit, and a surgical needle, wherein the surgical needle is accommodated in the needle driving unit to move with respect to the support, and a first suture from the surgical needle and a second suture from the support are entangled according to the movement, wherein the suture apparatus further comprises a fixing unit for fixing a tissue to be sutured to the support and releasing the fixation of the tissue.

In accordance with another aspect of the present invention, there is provided a suture apparatus comprising: a support, a needle driving unit, and a surgical needle, wherein the surgical needle is accommodated in the needle driving unit to move with respect to the support, and a suture from the surgical needle is used to suture a tissue according to the movement, wherein the suture apparatus further comprises a fixing unit for fixing the tissue to the support and releasing the fixation of the tissue.

### BEST MODE

### MODE FOR INVENTION

In the following detailed description, reference is made to the accompanying drawings that show, by way of illustration, specific embodiments in which the present invention may be practiced. These embodiments are described in sufficient detail to enable those skilled in the art to practice the present invention. It is to be understood that the various embodiments of the present invention, although different, are not necessarily mutually exclusive. For example, a particular feature, structure or characteristic described herein in connection with one embodiment may be implemented within other embodiments without departing from the spirit and scope of the present invention. In addition, it is to be understood that the location or arrangement of individual elements within each disclosed embodiment may be modified without departing from the spirit and scope of the present invention. The following detailed description is, therefore, not to be taken in a limiting sense, and the scope of the present invention is defined only by the appended claims, appropriately interpreted, along with the full range of equivalents to which the claims are entitled. In the drawings, like numerals refer to the same or similar elements throughout the several views.

Hereinafter, various preferred embodiments of the present invention will be described in more detail with reference to the accompanying drawings so that a person skilled in the art can easily practice the invention.

The overall configuration and detailed components of the suture apparatus in accordance with the present invention may be similar in many parts to those of the surgical suture apparatus disclosed in Korean Patent Application No. 2009-37128 as mentioned above. Thus, hereinafter, characteristic parts and those required to be described again or additionally in the configuration of the suture apparatus in accordance with the present invention will be largely described.

### Configuration of suture apparatus

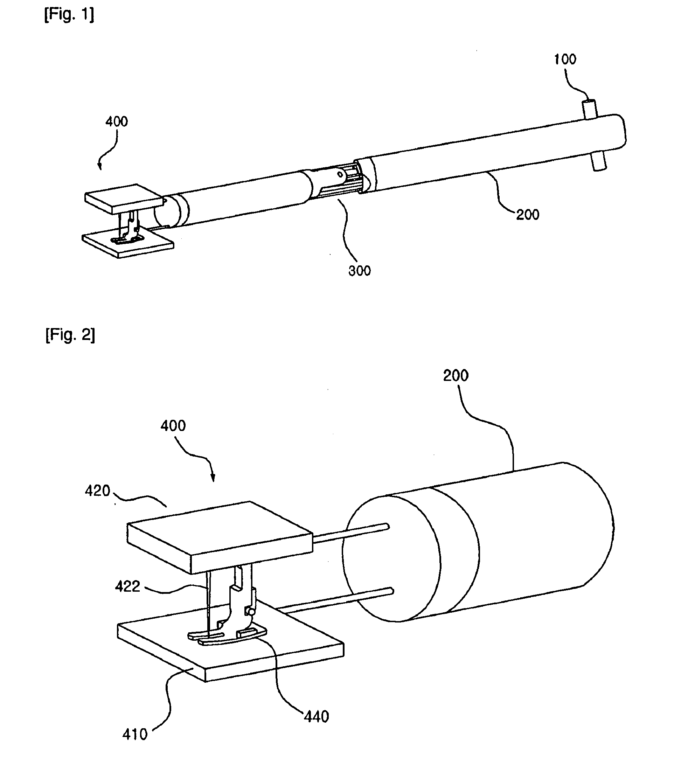

Fig. 1 is a drawing showing the overall configuration of a suture apparatus in accordance with an embodiment of the present invention.

Referring to Fig. 1, it can be seen that a suture apparatus in accordance with an embodiment of the present invention may be configured to include a coupling part 100, a shaft part 200, an operating part 300, and a suturing part 400.

In accordance with an embodiment of the present invention, when the suture apparatus is coupled to an external device (not shown) such as a surgical robot system having a robot arm or any other surgical systems, the coupling part 100 may perform a function of receiving a certain control signal or a certain control manipulation (e.g., a mechanical control manipulation) therefrom.

In accordance with an embodiment of the present invention, the shaft part 200 is an element for connecting the coupling part 100 and the suturing part 400. Here, one or more shafts may be provided with a joint element such as the operating part 300 to allow the suture apparatus to make a joint movement. The shafts may include wires or other control attachments for controlling the suturing part 400 as explained later. Further, the shaft part 200 may serve to physically support the suture apparatus.

In accordance with an embodiment of the present invention, the operating part 300 may be disposed at the ends of or between one or more shafts of the shaft part 200 to perform a function of allowing the suture apparatus to make a joint movement and a function of determining the position and/or direction of the suturing part 400 in response to a certain control signal from an external device or a certain control manipulation. The operating part 300 may enable the suturing part 400 and/or the shafts of the shaft part 200 to move in a pitch direction and/or a yaw direction as necessary.

Hereinafter, the suturing part 400 in accordance with various embodiments of the present invention will be described in detail.

Fig. 2 is a view showing the configuration of the suturing part 400 in accordance with an embodiment of the present invention.

As shown in Fig. 2, the suturing part 400 in accordance with an embodiment of the present invention may be configured to include a support 410 and a needle driving unit 420 including a surgical needle 422. Meanwhile, it is illustrated that the support 410 in accordance with the present invention is mainly a lower support 410, but it should be understood that the support 410 facing or being opposite to the surgical needle 422 may be disposed at an upper side or at a different side of the surgical needle 422.

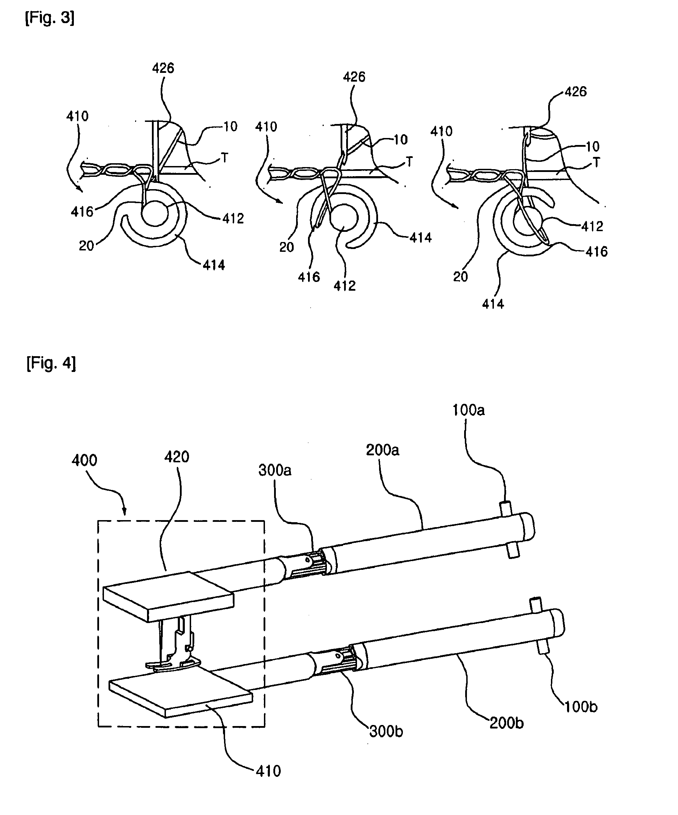

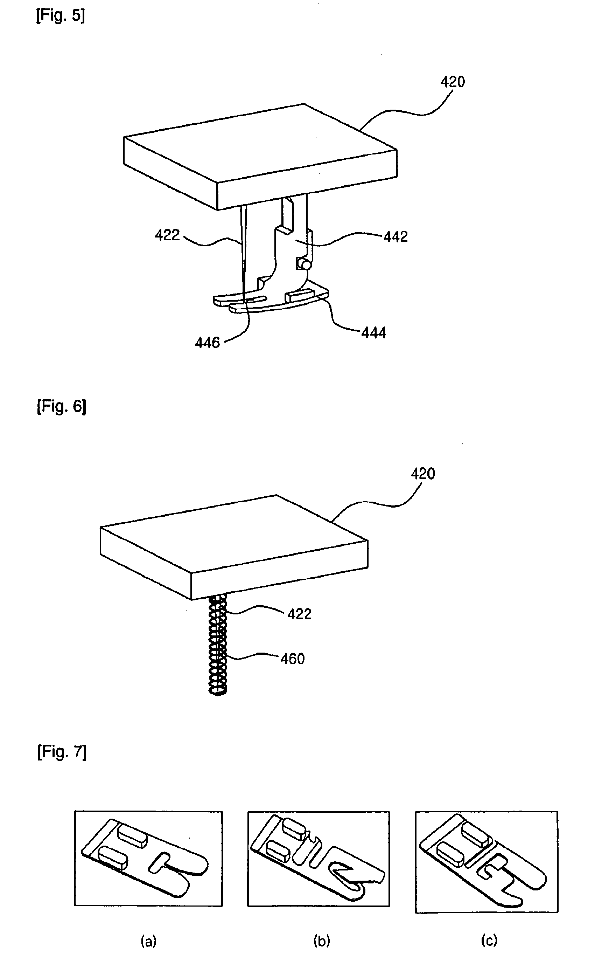

The support 410 in accordance with an embodiment of the present invention may basically perform a function of supporting tissues to be sutured. The needle driving unit 420 in accordance with an embodiment of the present invention may perform a function of operating the surgical needle 422 with respect to the support 410. Tissues may be sutured according to the movement of the surgical needle 422. The principle of the suturing will be described with reference to Fig. 3 as follows. Also, a detailed configuration of the needle driving unit 420 in accordance with the present invention will be described with reference to Fig. 5 and the like, as follows.

In various embodiments of the present invention, various principles which are the same as or similar to those discovered in various known sewing machines for make a suture by entangling a suture from the surgical needle 422 and a suture from the support 410 may be employed.

Fig. 3 is a drawing schematically showing an example of the principle of suturing of the suture apparatus in accordance with the present invention.

As shown in Fig. 3, the surgical needle 422 may perform a reciprocal movement with respect to the support 410 (i.e., with respect to the tissue T), and in this process, the surgical needle 422 may be holding a first suture 10, preferably by a portion close to a tip thereof.

Meanwhile, the support 410, supporting the tissue T, may provide a second suture 20. To this end, the support 410 may include an internal cylinder 412 therein. Also, the support 410 may further include an external cylinder 414. The internal cylinder 412 and the external cylinder 414 may be rotated together or separately. The second suture 20 may be disposed in the internal cylinder 412 and unwound therefrom. Meanwhile, the external cylinder 414 may include a certain recess 416.

This will be described in more detail. First, as shown in the left portion of Fig. 3, when the surgical needle 422, which moves with respect to the support 410 while holding the first suture 10, penetrates the tissue T, the first suture 10 held in the surgical needle 422 is caught in the recess 416 according to the rotation of the external cylinder 414 of the support 410 to form a certain ring. Subsequently, as shown in the middle portion of Fig. 3, a second suture 20, which is unwound from the internal cylinder 412 of the support 410 according to the rotation of the internal cylinder 412, may start to enter the ring of the first suture 10 which is increased according to the continued rotation of the external cylinder 414. In this case, the surgical needle 422, still holding the first suture 10, may move in a direction away from the support 410. Finally, as shown in the right portion of Fig. 3, when the surgical needle 422 nearly finishes a single reciprocal movement, a single rotation of the external cylinder 414 may be finished and the second suture 20 may enter the interior of the ring of the first suture 10, and accordingly, the first suture 10 and the second suture 20 may be entangled to suture the tissue T. In this case, the first suture 10 and the second suture 20 may be entangled but not tied. Hereafter, as the unitary suturing process described above is repeatedly performed, the overall suturing is achieved. Meanwhile, the first suture 10 and the second suture 20 may be preferably made of the same material.

In accordance with an embodiment of the present invention, the suturing part 400 may move the needle driving unit 420 in response to a control signal (from an external device) or a control manipulation. For example, as an operator manipulates the external device, the needle driving unit 420 may move along with the surgical needle 422 accommodated therein with respect to the support 410. In addition, for example, as the operator manipulates the external device, the needle driving unit 420 and/or the support 410 may be driven in a direction in which suturing is performed (in this respect, if necessary, the needle driving unit 420 and/or the support 410 may be configured to move as the body length of the shaft part 200 is elongated or contracted). Of course, the needle driving unit 420 and/or the support 410 may be driven in various directions other than the foregoing direction in order to realize suturing according to various suturing options as explained hereafter.

Meanwhile, it should be noted that a configuration in which options for suturing (e.g., 3-layer suture, 3-line suture, 4-line suture, zigzag suture, parallel suture, hemming suture, overlock suturing, and the like) are selectable, a configuration in which suturing proceeds in an automatic mode or a semiautomatic mode, a configuration in which the space of stitches and/or the suturing speed are determined or set, a configuration in which the overall configuration or dimensions of the suture apparatus are changed as necessary so as to be appropriate for various surgical operations such as an invasive surgery, or the like may be adopted for the suture apparatus in accordance with the present invention, with reference to the description of Korean Patent Application No. 2009-37128.

Further, it should be noted that a configuration in which a rail (not shown) is applied to the needle driving unit 420, a configuration in which a rotating unit (not shown) is applied to the suturing part 400, or the like may be adopted for the suture apparatus in accordance with the present invention with further reference to the description in Korean Patent Application No. 2009-37128.

Fig. 4 is a drawing showing the overall configuration of a suture apparatus in accordance with another embodiment of the present invention. As shown in Fig. 4, the suture apparatus in accordance with the present invention may have a so-called separation type configuration. That is, the suture apparatus may be configured to include a first coupling part 100a, a second coupling part 100b, a first shaft part 200a, a second shaft part 200b, a first operating part 300a, a second operating part 300b, and a suturing part 400.

In accordance with the present embodiment, as shown in Fig. 4, an upper portion of the suturing part 400 including the needle driving unit 420 as described below may form a separated module of the suture apparatus along with the first coupling part 100a, the first shaft part 200a, and the first operating part 300a, and as shown in Fig. 4, the support 410 corresponding to a lower portion of the suturing part 400 may form another separated module of the suture apparatus along with the second coupling part 100b, the second shaft part 200b, and the second operating part 300b. Here, the first coupling part 100a, the first shaft part 200a, and the first operating part 300a may be separated from the second coupling part 100b, the second shaft part 200b, and the second operating part 300b.

The respective modules of the suture apparatus having the separation type configuration in accordance with the present embodiment may approach a tissue to be sutured via various paths. Also, the joint movement in each module can be freer, and the respective modules can be controlled by different external devices. The above suture apparatus may be advantageous in case of suturing a tubular internal organ or performing suturing by using a surgical port.

Korean Patent Application No. 2009-37128 may be further referred with respect to the modified configuration of the suture apparatus in accordance with the present invention as described above with reference to Fig. 4.

### Configuration of fixing means

In accordance with an embodiment of the present invention, the suturing part 400 of the suture apparatus may include a fixing means (i.e., fixing unit) for ensuring effective suturing.

Fig. 5 is a drawing illustrating the configuration of a fixing unit 440 in accordance with an embodiment of the present invention.

Referring to Fig. 5, the fixing unit 440 may be configured to include a fixing rod 442 and a pressing plate 444. The fixing unit 440 may perform a function of fixing a tissue to be sutured with respect to the support 410 or releasing the fixation of the tissue. The presence of the fixing unit 440 can prevent a problem in which the tissue deviates from its proper position on the support 410 in the course of suturing, and can stably perform automatic suturing.

First, the fixing rod 442 allows the pressing plate 444 connected thereto to press the tissue so as to be fixed to the support 410 or release the fixation of the tissue.

In accordance with an embodiment of the present invention, the fixing rod 442 may be accommodated in the needle driving unit 420 and moved along with the needle driving unit 420 (namely, further along with the surgical needle 422) to allow the pressing plate 444 to operate to fix the tissue to the support 410 or release the fixation of the tissue in the process of suturing (in particular, in the process in which the surgical needle 422 penetrates the tissue and is removed from the tissue). The moving direction of the fixing rod 442 when the tissue is to be fixed may be a direction from the needle driving unit 420 to the support 410. Of course, the moving direction of the fixing rod 442 when the fixation of the tissue is to be released may be a direction opposite to the foregoing direction. The fixing rod 442 may be made of an elastic material or may include an elastic member such that the length thereof can be increased or decreased. Such a configuration may be useful in case the pressing plate 444 presses the tissue by elastic force corresponding to the thickness of the tissue.

In another embodiment of the present invention, the fixing rod 442 may be configured to be accommodated in the needle driving unit 420 and moved along with the needle driving unit 420 such that at least a portion thereof is elongated to come to a position at which it can appropriately fix the tissue with the pressing plate 444. Of course, when the pressing plate 444 releases the fixation as mentioned above, at least a portion of the fixing rod 442 may be contracted. In many cases, preferably, the cycle of the movement of the fixing rod 442 may be substantially equal to that of the needle driving unit 420 (that is, the cycle of the movement of the surgical needle 422). Also, even in accordance with the present embodiment, in order to apply more pressing force to a thicker tissue, the fixing rod 442 preferably may be made of an elastic material or may include an elastic member such that the length thereof may be increased or decreased.

Next, the pressing plate 444 may serve to directly press the tissue to be fixed to the support 410 or release the fixation of the tissue. The pressing plate 444 may be removably attached to the fixing rod 442. The pressing plate 444 may be preferably configured to be in surface-contact with the tissue to stably fix the tissue to the support 410. Further, in order to increase frictional force of the pressing plate 444 with respect to the tissue, a surface of the pressing plate 444 facing the tissue may have recesses and protrusions formed thereon (not shown).

Meanwhile, one or more openings 446 may be provided in the pressing plate 444 in order to provide a space for allowing the movement of the surgical needle 422. As shown in Fig. 5, the openings 446 may have a shape similar to the most common claws used in a known sewing machine, but as shown in Fig. 7, it may have a shape similar to various different types of claws used in a known sewing machine (Fig. 7 illustrates (a) a pressing plate for zigzag suture, (b) a pressing plate for hemming suture, and (c) a pressing plate for overlock suture). The operator may select one of various different types of pressing plates 444 having the openings 446 formed in various patterns, thereby using the most appropriate pressing plate 444 for one of the various different suture options as mentioned above which the operator could have already selected. In this case, a replacement of the pressing plate 444 or a removal of the pressing plate 444 with respect to the fixing rod 442 as necessary may be performed automatically or according to a manual manipulation of an experienced operator.

Meanwhile, even when the surgical needle 422 is removed from the tissue along with the fixing unit 440 in accordance with the present invention, a spring 460 may be used to prevent the tissue from being released together with the surgical needle 422.

Fig. 6 is a drawing illustrating the spring 460 in accordance with an embodiment of the present invention. As illustrated, the spring 460 may be accommodated in the needle driving unit 420 with the surgical needle 422 being placed at the center thereof (of course, the disposition of the spring 460 is not limited thereto). Preferably, the spring 460 is configured to have sufficient elasticity to help the removal of the surgical needle 422 from the tissue.

While embodiments of the invention has been shown and described, it will be understood by those skilled in the art that various changes and modifications may be made without departing from the spirit and the scope of the invention as defined in the following claims.

## Claims

1. A suture apparatus comprising:
a support;
a needle driving unit; and
a surgical needle,
wherein the surgical needle is accommodated in the needle driving unit to move with respect to the support, and
a first suture from the surgical needle and a second suture from the support are entangled according to the movement,
wherein the suture apparatus further comprises a fixing unit for fixing a tissue to be sutured to the support and releasing the fixation of the tissue.

2. The suture apparatus as claimed in claim 1, wherein the fixing unit includes a fixing rod and a pressing plate.

3. The suture apparatus as claimed in claim 1, wherein the fixing rod is made of an elastic material or includes a certain elastic member.

4. The suture apparatus as claimed in claim 2, wherein the fixing rod is configured such that at least a portion thereof performs elongation and contraction movements.

5. The suture apparatus as claimed in claim 4, wherein a cycle of the movement of the fixing rod is substantially equal to that of the surgical needle.

6. The suture apparatus as claimed in claim 2, wherein the pressing plate is removably attached to the fixing rod.

7. The suture apparatus as claimed in claim 2, wherein the pressing plate has an opening.

8. The suture apparatus as claimed in claim 7, wherein the opening is formed in a pattern according to an option selected for suturing.

9. The suture apparatus as claimed in claim 2, wherein the pressing plate has recesses and protrusions on a surface facing the tissue.

10. The suture apparatus as claimed in claim 1, further comprising a spring to help the removal of the surgical needle from the tissue.

11. A suture apparatus comprising:
a support;
a needle driving unit; and
a surgical needle,
wherein the surgical needle is accommodated in the needle driving unit to move with respect to the support, and
a suture from the surgical needle is used to suture a tissue according to the movement,
wherein the suture apparatus further comprises a fixing unit for fixing the tissue to the support and releasing the fixation of the tissue.
